(19) Europäisches Patentamt / European Patent Office / Office européen des brevets

(11) **EP 2 751 775 B1**

(12) **EUROPEAN PATENT SPECIFICATION**

(45) Date of publication and mention
of the grant of the patent:
**06.07.2016 Bulletin 2016/27**

(51) Int Cl.:
*G06T 3/00* (2006.01)          *G06T 11/00* (2006.01)
*G06T 1/00* (2006.01)          *A61F 2/00* (2006.01)
*G09B 21/00* (2006.01)          *A61N 1/36* (2006.01)

(21) Application number: **12827966.8**

(22) Date of filing: **29.08.2012**

(86) International application number:
**PCT/AU2012/001006**

(87) International publication number:
**WO 2013/029097 (07.03.2013 Gazette 2013/10)**

(54) **SYSTEM AND METHOD FOR PROCESSING SENSOR DATA FOR THE VISUALLY IMPAIRED**

SYSTEM UND VERFAHREN ZUR VERARBEITUNG VON SENSORDATEN FÜR SEHBEHINDERTE

SYSTÈME ET PROCÉDÉ DE TRAITEMENT DE DONNÉES DE CAPTEUR POUR DES PERSONNES MALVOYANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.08.2011 US 201161529071 P**

(43) Date of publication of application:
**09.07.2014 Bulletin 2014/28**

(73) Proprietor: **Monash University**
**Clayton, Victoria 3800 (AU)**

(72) Inventors:
• **LUI, Wen Lik Dennis**
**Clayton**
**Victoria 3168 (AU)**
• **BROWNE, Damien**
**Glen Waverley**
**Victoria 3150 (AU)**
• **DRUMMOND, Tom**
**Viewbank**
**Victoria 3084 (AU)**
• **LI, Wai Ho**
**Ringwood North**
**Victoria 3134 (AU)**
• **KLEEMAN, Lindsay**
**Vermont South**
**Victoria 3133 (AU)**

(74) Representative: **Meissner Bolte Partnerschaft mbB**
**Hollerallee 73**
**28209 Bremen (DE)**

(56) References cited:
WO-A2-2008/109771      GB-A- 2 477 431
KR-A- 20030 015 936      KR-A- 20090 105 531
US-A1- 2005 168 569      US-A1- 2010 220 176
US-A1- 2011 164 163      US-B1- 6 658 299

EP 2 751 775 B1

**Description**

The present invention relates to visual aids, including visual prostheses ('bionic eyes'), and particularly to methods and apparatus for processing images and other sensor inputs to provide improved rendering of visual information to a user.

Implanted visual aids based upon electrical stimulation of still-functional parts of the visual system (also known as 'bionic eyes') hold promise as a method to alleviate visual impairment of varying degrees, up to and including total blindness.

One prior art approach to the development of bionic visual aids involves stimulating the retina of the subject, in order to harness the natural visual pathways (i.e. optic nerves) to take information to the visual cortex of the brain.

In an alternative approach, the visual cortex may itself be stimulated in order to generate visual percepts.

In fact, it has been found that phosphenes (i.e. percepts in the form of bright dots of light) may be elicited through electrical stimulation of various portions of the visual pathway. In healthy individuals, visual signals are carried from the retina to the primary visual cortex (V1), via the optic nerve and lateral geniculate nucleus (LGN). As visual signals congregate at V1 before diverging to higher-level processing, electrical stimulus can be injected anywhere between the retina and V1 to elicit phosphenes and effectively override the signals from earlier parts of the visual system. Proposed visual prothesis operate based upon this principle to bypass damaged parts of the visual pathway, replacing missing visual signals with bionic vision signals comprising artificial electrical stimulation.

Proposed visual prostheses generally rely on video input obtained from a head-mounted camera or in-eye imager. As a practical matter, viable visual prostheses have limited resolution. Accordingly, in prior art approaches video signals are down-sampled to produce corresponding low resolution images. These images must be converted into a suitable corresponding pattern of electrical stimulus, via a neuromorphic coding process. An electrode array, e.g. in the retina or the visual cortex, conveys the electrical stimulus to the visual pathway. Advantageously, it has been found that predictable phosphene behaviour may be achieved via electrode arrays implanted into the retina or primary visual cortex (V1). Either form of implant allows the generation of a grid of phosphenes that appears similar to a low-resolution digital image.

While such visual prostheses are promising, the limited resolution remains a problem. In this context, resolution refers both to the number of pixels, and the distinct levels of intensity (or brightness) that can be represented by each pixel. By way of example, it is presently believed that a practical cortical implant may be developed using available technologies and bio-compatible materials, capable of generating a grid of 625 phosphenes, equivalent to a 25 x 25 pixel array, in which each pixel is binary, i.e. on or off, black or white.

The extreme downsampling involved in reducing captured visual images to such low resolution suffers from the problem of significant loss of salient information. Important visual information, such as the locations of, and distinctions between, edges, planes, distinct objects, patterned surfaces, and so forth, are typically lost in the 'flattened' low-resolution images.

International Patent Application Publication No. WO2008/109771 discloses a saliency-based apparatus and method for use with a visual prosthesis, in which the subject's attention is focussed upon regions within an input image that are notably different from their surroundings.

UK Patent Application Publication No. GB2477431 discloses a vision substitution system for communicating audible and/or tactile representations of features in visual images to vision-impaired subjects.

US Patent No 6,658,299 discloses an artificial vision system in which image processing methods are employed to invert light and dark regions, and to enhance edges, in visual images.

It is, accordingly, an object of the present invention to provide visual processing systems, apparatus and methods that are better able to mediate salient visual representations under constraints such as limited spatial and intensity resolution.

In one aspect, the present invention provides a prosthetic processing apparatus , the apparatus comprising: a visual sensor configured to output two-dimensional image information as physical information of a spatial field; at least one additional sensor configured to output physical information other than two-dimensional image information of the spatial field an output interface coupled to a sensory input device which is configured to apply a signal to a visual sensory pathway of the visually impaired subject; and a processor operatively coupled to the sensors and to the output interface, and which is configured to: receive the physical information of the spatial field from the sensors; process the received information to identify one or more salient features of a predetermined category within the spatial field; generate a transformed representation of the spatial field in which each identified salient feature is represented in a symbolic form subject to predetermined fidelity constraints imposed by capability of the sensory input device, said transformed representation being an image; and output the transformed representation to the sensory input device via the output interface.

The present inventors have recognised that the truncation of salient visual information via a downsampling process is a fundamental limitation of previously proposed low-resolution visual processing systems, such as bionic vision processors. In particular, downsampling results in useful visual images only in constrained environments, which are simple, predictable, and high in contrast. Advantageously, therefore, embodiments of the present invention utilise 'intelligent' processing algorithms that are able to extract and represent selected salient visual features from sensor information relating to the spatial field, and transform these salient features into symbolic forms that are able to convey meaningful detail to the subject. The inventors have applied the term 'Transformative Reality' (TR) to this novel concept.

In general the terms 'symbol' and 'symbolic' refer to representations that 'stand in for' something else. In the context of TR, and as used in this specification, a 'symbol' or a 'symbolic form' refers more specifically to a representation communicable to the subject via the output interface and the sensory input device to be received in the sensory pathway in a form that enables the subject to distinguish the salient features corresponding with the representation. A symbol may be, for example, an intelligible pattern of dots or pixels communicable to the subject as phosphenes elicited by stimulation of the visual pathways. Such stimulation may be invasive (e.g. via a retinal or cortical implant), or non-invasive (e.g. via a head-mounted display, for users with some residual visual function). Alternatively, a symbol may be an intelligible pattern of sounds communicable to the subject via the aural pathways, such as through headphones or a cochlear implant. Symbolic representations may be communicated to a subject in general via any suitable sensory modality.

In embodiments of the invention, the predetermined categories of salient features may include edges, plane surfaces, human faces and/or bodies, and other distinct visual elements or objects appearing within the spatial field.

In embodiments of the invention, the processor comprises a microprocessor with associated memory, the memory containing executable instructions which, when executed by the microprocessor, cause the microprocessor to apply the transformative algorithms to the received physical information.

In embodiments of the invention, the output interface is coupled to a cortical implant arranged to apply electrical stimulation to the user's visual cortex corresponding with the representation of the transformed image. In other embodiments, the output interface is coupled to a retinal implant arranged to apply electrical stimulation to the user's retina corresponding with the representation of the transformed image. In all of these embodiments, the invention provides for an improved visual prosthesis.

In alternative embodiments, the output interface may be coupled to sensory input devices configured to apply signals to sensory pathways other than, or in addition to, the visual pathway. For example, an aurally-intelligible representation of the spatial field may be generated comprising either a mono- or stereophonic audible signal in which salient features may be represented symbolically by signal properties such as frequency, volume and apparent origin of sounds, i.e. stereo imaging or 'soundstage'.

In some embodiments of the invention, the additional sensor comprises one or more of a depth sensor (e.g. an active infrared depth camera, stereo camera, or time-of-flight camera) and an accelerometer.

In embodiments comprising a depth sensor, the processor may be configured to apply a structural edge-detection algorithm whereby physical information received from the depth sensor is processed to identify locations at which discontinuities in depth are detected. Discontinuities include 'sharp' edges, such as may occur at the margins of solid objects such as items of furniture, and 'softer' edges, such as creases, ripples, corrugations and the like, such as may occur in flexible items, such as clothing and fabrics. These locations at which discontinuities in depth are detected are rendered as contrasting pixels in the transformed image. In a visual prosthesis, the contrasting pixels correspond with phosphenes generated within an artificially-induced field of vision of a user having a visual prosthetic implant.

In some embodiments, the processor is configured to apply a face detection algorithm to two-dimensional image information output from the visual sensor in order to identify the location of faces within the image. The processor may additionally apply a body detection algorithm whereby physical information received from a depth sensor is processed to identify physical configuration of human bodies associated with located faces. Advantageously, this form of face and body detection enables human subjects within the spatial field to be rendered symbolically with enhanced clarity, and facilitates the representation within the transformed image of visual cues normally taken for granted when interacting with human beings, including the pose of a subject's face and body (e.g. location, orientation, expression, posture and gestures).

In some embodiments, a face detection algorithm comprises a boosted Haar cascade algorithm.

Once one or more faces have been identified, embodiments of the invention perform body detection by conducting a proximity search of depth sensor information to identify features falling within a specified volume in the vicinity of located faces.

In some embodiments, the locations at which faces are detected are rendered symbolically as facial icons or avatars constructed from contrasting pixels in the transformed image. Similarly, the configuration of human bodies is rendered symbolically as corresponding contrasting pixels in the transformed image.

In some embodiments comprising an accelerometer, the processor is configured to estimate a direction of gravity based upon physical information received from the accelerometer, whereby a spatial orientation of information received from other sensors, such as received two-dimensional image information and/or received depth information, is determined.

Embodiments may comprise a depth sensor and an accelerometer, and the processor configured to apply a ground plane detection algorithm whereby physical information received from the depth sensor is processed along with physical information received from the accelerometer in order to identify locations corresponding with a contiguous substantially horizontal plane surface with the spatial field.

Advantageously, ground plane detection enables the consistent symbolic rendering of flat horizontal surfaces, such as floors, independent of patterning, textures and other objects located within the spatial field, significantly enhancing the potential for a user to navigate successfully based upon a visual representation of the transformed image.

In an embodiment, the ground plane detection algorithm comprises:

generating a plane hypothesis corresponding with a hypothetical plane disposed at a predetermined elevation relative to the depth sensor;

testing the plane hypothesis by comparing a distance measure of points within the spatial field detected by the depth camera with points on the hypothetical plane; and

accepting the plane hypothesis in the event that the comparison establishes a sufficiently close correlation between the detected points and the points on the hypothetical plane, such as having a sufficient number of detected points within a distance threshold of the hypothetical plane (inliers).

In one embodiment, the ground plane detection algorithm further comprises generating multiple plane hypotheses corresponding with hypothetical planes disposed at a plurality of predetermined elevations relative to the depth sensor; and accepting the plane hypothesis having the closest correlation between the detected points and the points on the hypothetical plane.

In some embodiments, physical information received from the accelerometer is used in particular for determining a direction normal to the hypothetical ground plane, and is subject to sensor noise, user movement and imperfections of the physical ground plane, amongst other potential sources of uncertainty. In embodiments providing for improved ground plane detection, the algorithm includes estimating the location of the horizontal plane surface by determining an improved plane estimate by applying an iterative method based upon sampling of depth sensor information corresponding with points within the accepted hypothetical plane. In at least one implementing approach the iterative method is random sample consensus (RANSAC).

In embodiments of the invention, the locations corresponding with the estimated horizontal plane surface are rendered symbolically as contrasting pixels in the transformed image.

In some embodiments, the processor is configured to apply a blending algorithm to generate a transformed image comprising elements of corresponding images produced by two or more transformative algorithms. Blending may be performed automatically, and/or under user control, in order to render multiple salient features within the representation of the transformed image. Advantageously, the blending algorithm assigns a precedence to the images produced by the two or more transformative algorithms, in a manner that results in the most effective symbolic presentation of salient information. The order of precedence may be intelligently adapted by the blending algorithm based on the type of symbols being presented to a human user.

For example, in some embodiments the transformative algorithms comprise two or more of a ground plane detection algorithm, a structural edge-detection algorithm, and a face-and-body detection algorithm. Rendering of face and body images has precedence over rendering of ground plane images, which in turn has precedence over rendering of structural edges. This is because detected human beings will generally be in the foreground of the spatial field, rendering of the ground plane is important to enable safe navigation within the spatial field, while structural edged will typically be located adjacent to, or outside, the region occupied by the ground plane,

In another aspect, the present invention provides a visual processing method comprising:

receiving information from a plurality of sensors configured to capture and output physical information of a spatial field, wherein the plurality of sensors comprises: a visual sensor configured to output two-dimensional image information of a spatial field; and at least one additional sensor configured to output physical information other than two-dimensional image information of the spatial field; processing the received information to identify one or more salient features of a predetermined category within the spatial field; generating a transformed representation of the spatial field in which each identified salient feature is represented in a symbolic form subject to predetermined fidelity constraints imposed by capability of a sensory input device configured to apply a signal to a visual sensory pathway of a visually impaired subject, said transformed representation being an image; and outputting the transformed representation to the sensory input device.

In some embodiments, the method is employed within a visual prosthetic system, wherein the output representation is communicated to a visual prosthetic implant, such as a cortical implant arranged to apply electrical stimulation to the user's visual cortex or a retinal implant arranged to apply electrical stimulation to the user's retina. However, in alternative embodiments, the output representation may be communicated to sensory input devices configured to apply signals to sensory pathways other than, or in addition to, the visual pathway.

In some embodiments, the method comprises applying a structural edge-detection algorithm whereby physical information received from a depth sensor is processed to identify locations at which discontinuities in depth are detected.

In some embodiments the method comprises applying a face detection algorithm to two-dimensional image information received from a visual sensor (e.g. a digital camera or CCD array) to identify the location of faces within the image, and applying a body detection algorithm whereby physical information received from the depth sensor is processed to identify physical configuration of human bodies associated with located faces.

Applying a face detection algorithm may comprise employing boosted Haar cascades.

In some embodiments, applying a body detection algorithm comprises conducting a proximity search to identify features within the information received from the depth sensor falling within a specified volume in the vicinity of located faces.

In some embodiments, the method comprises estimating a direction of gravity based upon physical information received from an accelerometer, whereby a spatial orientation of the received two-dimensional image information is determined.

Embodiments of the method may comprise applying a ground plane detection algorithm whereby physical information received from a depth sensor is processed along with physical information received from an accelerometer in order to identify locations corresponding with a contiguous substantially horizontal plane surface within the spatial field.

Embodiments of the method may further comprise applying a blending algorithm to generate a transformed image comprising elements of corresponding images produced by two or more transformative algorithms, such as a ground plane detection algorithm, a structural edge detection algorithm, and a face-and-body detection algorithm.

In another aspect, the present invention provides a visual processing apparatus comprising:

a visual sensor configured to output two-dimensional image information of a spatial field;
at least one additional sensor configured to output physical information other than two-dimensional image information of the spatial field;
a processor operatively coupled to the sensors to receive the image information from the visual sensor and the physical information from the additional sensor, and configured to apply one or more transformative algorithms to combine the image information and physical information to produce a transformed image of the spatial field, wherein the transformed image comprises a visual rendering of selected salient features that are not identifiable based on processing of the two-dimensional image information alone, and wherein the transformed image is subject to predetermined fidelity constraints; and
an output interface, operatively coupled to the processor, wherein the processor is further configured to output a representation of the transformed image via the output interface.

Further features and benefits of the invention will be apparent to the persons skilled in the art from the following description of preferred embodiments, which are provided by way of example only, and without limitation to the general scope of the invention as described in the foregoing statements, and defined in the claims appended hereto.

## BRIEF DESCRIPTION OF THE DRAWINGS

Embodiments of the invention will now be described with reference to the accompanying drawings in which:

Figure 1 is a block diagram illustrating a general structure of a Transformative Reality framework according to an embodiment of the invention;
Figure 2 is a block diagram of a microprocessor-based apparatus embodying the framework of Figure 1;
Figure 3 is a block diagram illustrating an exemplary Transformative Reality system for visual prostheses, according to the invention;
Figure 4 is a flowchart illustrating a structural edge-detection algorithm according to an embodiment of the invention;
Figure 5 is an illustration of the results of application of the algorithm represented in Figure 4;
Figure 6 is a flow-diagram illustrating a ground plane detection algorithm according to an embodiment of the invention;
Figure 7 illustrates the results of application of the algorithm represented in Figure 6;
Figure 8 is a flow-diagram illustrating a face-and-body detection algorithm embodying the invention;
Figure 9 illustrates the results of application of the algorithm represented in Figure 8;
Figure 10 is a flowchart illustrating an automatic blending algorithm embodying the invention;
Figure 11 illustrates blending of two transformed images according to the algorithm represented in Figure 10; and
Figure 12 illustrates the blending of three transformed images according to the algorithm represented in Figure 10.

**DETAILED DESCRIPTION**

Embodiments of the present invention employ a novel 'Transformative Reality' (TR) conceptual framework to optimise the saliency of information presented through visual prostheses. The TR framework is not limited to simple and direct transformations of visual data from camera to implant, such as the downsampling applied in prior art approaches, and comprises real-time transformations of sensor data, including data from non-visual sensors, to generate a mixture of symbolic and structural content that is registered to the real world. The inventors' aim is to employ the TR concept to bypass limitations in human sensing by rendering sensor data in ways that are more easily understood by the user given his or her constraints. In the case of embodiments comprising a visual prosthesis, the framework enhances the effectiveness of the limited resolution display.

While exemplary embodiments described herein are directed to applications in prosthetic vision, the novel TR framework is also applicable to other sensing modalities. For example, the framework includes sensory substitution devices that uses sounds and touch to represent the visual world. Furthermore, the TR framework may find other applications, such as in machine vision. Accordingly, it will be understood by persons skilled in the relevant art that the exemplary embodiments are not limiting of the scope of the invention.

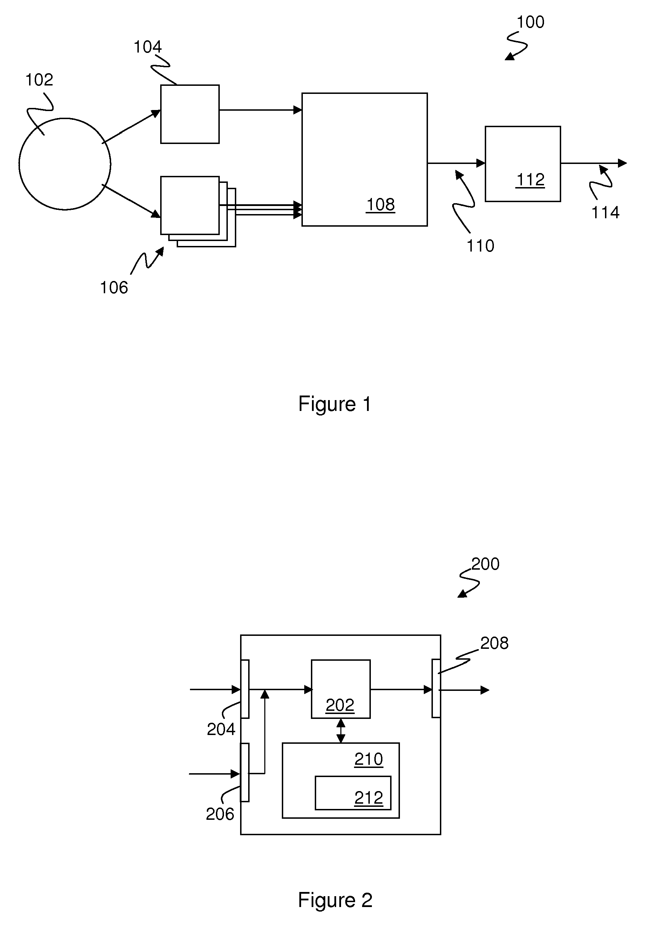

The general structure of the TR framework 100 is illustrated in the block diagram of Figure 1. It is an object of TR to intelligently render sensor data in real time into virtual content that is then presented to the user.

The TR framework, which may be embodied within a microprocessor-based apparatus 200, as discussed below with reference to Figure 2, receives input from the outside world 102 via at least one sensor 104, and optionally a number of additional sensors 106.

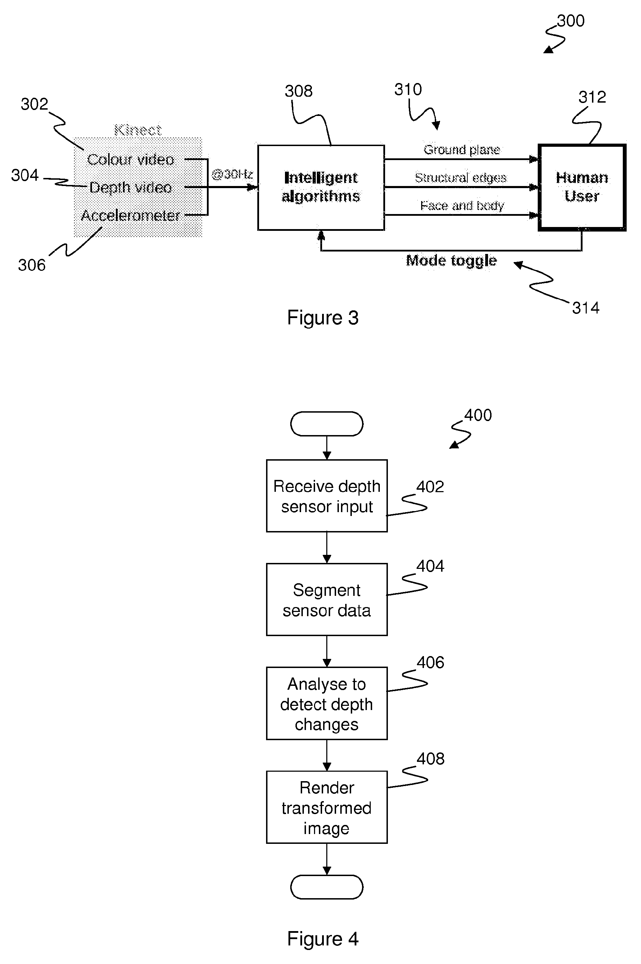

In an exemplary embodiment, a visual sensor 104, such as a digital camera or CCD array, is configured to output two-dimensional image information of a spatial field within its field-of-view of the world 102. One or more additional sensors 106 are configured to output physical information other than two-dimensional image information of the spatial field. For example, as discussed further below with reference to Figure 3, in an exemplary embodiment of the invention the additional sensors 106 comprise an accelerometer and a depth sensor.

Outputs from the sensors 104, 106 are input to one or more transformative algorithms 108, which process the received sensor information and generate a transformed representation of the spatial field within the world 102. The transformed representation is an image comprising a visual rendering 110 of selected salient features that are represented in a symbolic form, in particular to mitigate predetermined fidelity constraints, such as the limited resolution of prosthetic vision devices. Optionally, additional 'virtual content' 112 may be added to, or superimposed upon, the visual rendering 110.

Finally, the transformed representation is output 114. This representation may take the form, for example, of an array of pixels corresponding with phosphenes to be generated by electrical stimulation of the visual pathway of a user. At a lower level, the representation may be the driving electrical stimulation signals themselves, i.e. following suitable neuromorphic coding.

Advantageously, the TR framework 100 embodies three concepts that help enhance the world rendered to the user through low resolution bionic vision compared to traditional bionic vision.

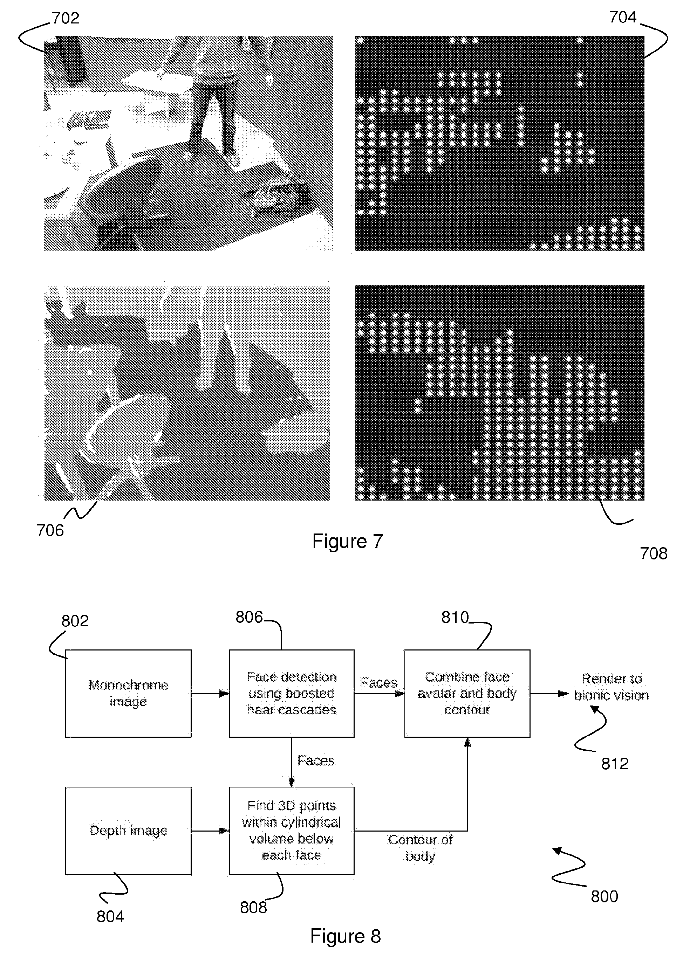

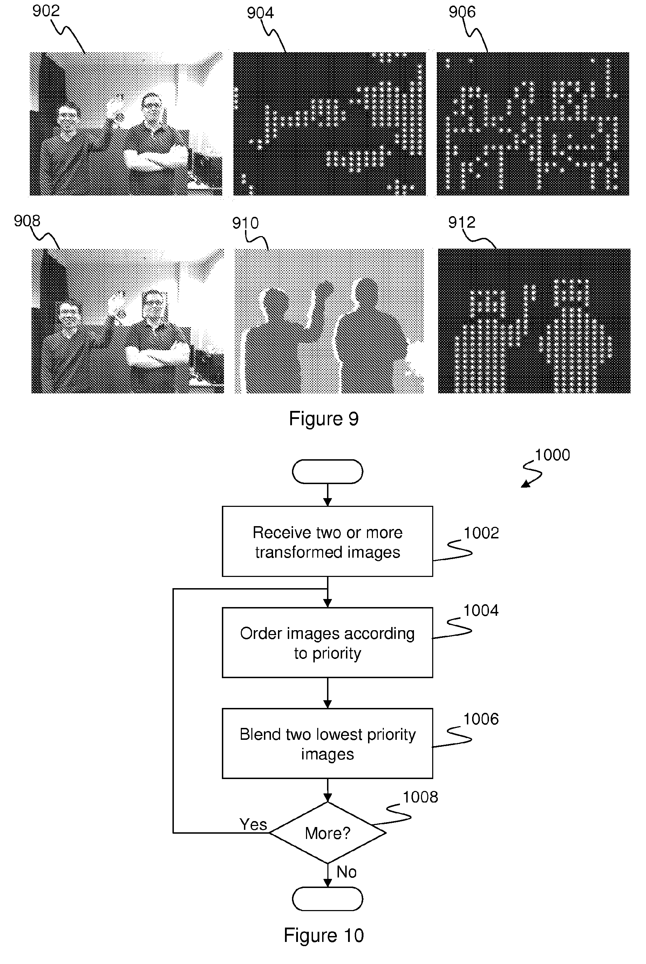

Firstly, sensor data is transformed in real-time into symbolic and structural content instead of the direct mapping imposed by downsampling. This enables the presentation of symbolic content registered to salient structural content. Everything conveyed by TR is virtual content, and real-world sensor data is naturally transformed into well-registered symbolic and structural content. One example of this is the 'face and body' rendering discussed below with reference to Figures 8 and 9, in which a low resolution avatar or icon (much like a bitmap font) is used to represent frontal faces while also highlighting a person's body as a filled region below the face icon. This combination of symbolic and structural content, registered to the real world, provides salient information that is not available via traditional bionic vision due to limited spatial resolution and the sensing requirement of strong visual contrast. In addition, the allowance for indirect representations of the world also allows multiple modes of transformation for different visual tasks.

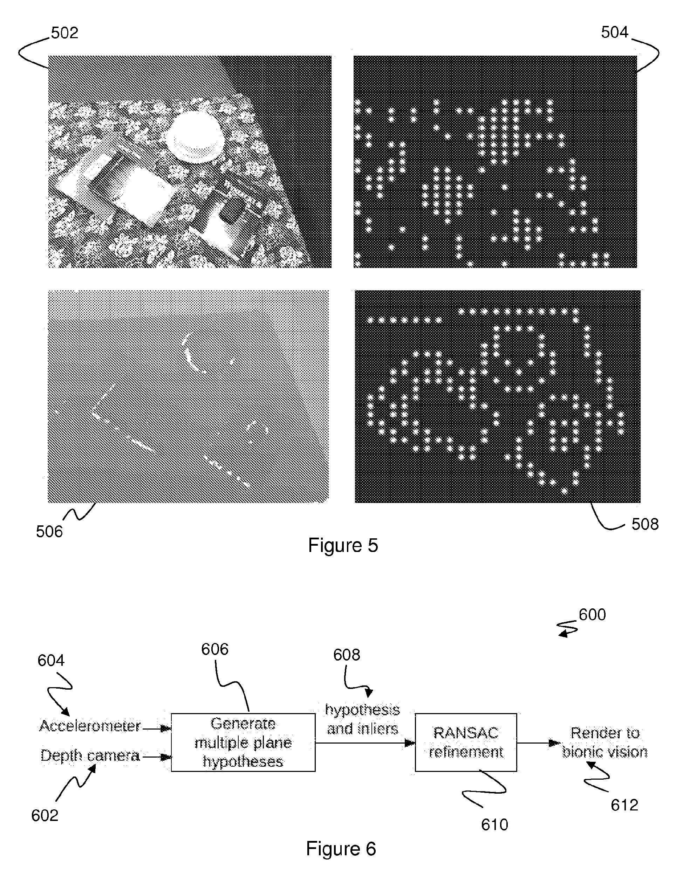

Secondly, TR may employ multiple sensors, 104,106, to provide input data. Considering the desirability of optimising the visual information presented through bionic vision, the use of additional sensors can greatly enhance the quality of the rendering by making sure that salient information is sensed and ensuring that this information is well-registered with the world around the user. The use of non-visual sensors 106 may provide a visual rendering not otherwise possible using vision sensors. Advantageously, sensors are provided that are best suited to a selected mode of transformation or for specific visual tasks. An example of this principle is the 'ground plane' rendering described below with reference to Figures 6 and 7, which employs a depth camera and accelerometer to generate a low-resolution rendering of the ground plane to represent navigational clearance of a complex scene to the user. This is a difficult task in complex environments when using direct visual representation due to the low resolution of bionic vision and the unreliable three-dimensional information available from a single video camera. As a further example, the 'structural edges' rendering described below with reference to Figures 4 and 5 employs a depth image to render the three-dimensional edges of a scene in real time.

Thirdly, systems embodying the TR framework may provide a real-time interface that allows user control of how sensor data is rendered into virtual content. This effectively allows the user to adjust parameters of the TR system such as the mode of transformation applied to sensor data. The TR system may also be configured to intelligently blend mode outputs, using methods such as that described below with reference to Figures 10-12.

Figure 2 is a block diagram illustrating a microprocessor-based apparatus 200 implementing the TR framework.

The exemplary apparatus 200 includes a microprocessor 202, which is operatively associated with sensor data inputs 204, 206. The microprocessor 202 is also operatively associated with an output interface 208, via which transformed image data may be output.

The microprocessor 202 is further associated with a memory device 210, such as random access memory, read only memory, and/or other forms of volatile and non-volatile memory device.

In exemplary embodiments of the invention directed to bionic vision systems, the apparatus 200 may be an image processing system that is worn on the person of the user, and arranged to receive inputs from various sensors, including an image sensor, such as a digital camera or CCD device, along with other physical sensor devices, and to generate output signals that are conveyed to a prosthetic implant, which may be located, for example, in the user's retina, or in the visual cortex. As such, the microprocessor-based apparatus 200 may be a low-power, battery-operated unit, having a relatively simple hardware architecture along the lines illustrated in the block diagram of Figure 2. However, the apparatus 200 may be implemented in a variety of ways, including by processing performed on a general-purpose computer, such as a laptop or desktop computer, and accordingly the absence of additional hardware details in Figure 2 should not be taken to indicate that other standard components may not be included within a practical embodiment of the invention.

The memory device 210 comprises, in use, a body of stored program instructions 212. These program instructions, and other volatile and/or non-volatile contents of the memory 210, are executable by the microprocessor 202, and are adapted such that the apparatus 200 is configured to perform various processing functions, and to implement various algorithms, such as are described below, and particularly with reference to Figures 4 to 12.

Figure 3 is a block diagram 300 illustrating the general architecture of an exemplary Transformative Reality system, which may be implemented via the microprocessor-based apparatus 200, suitable for visual prostheses.

The system 300 includes a colour video image sensor 302, a depth sensor 304, and an accelerometer 306. In an exemplary embodiment, which has been implemented using a Microsoft™ Kinect™ sensor, the depth sensor 304 is an infrared depth camera, and the accelerometer 306 is a three-axis sensor.

The information output from the sensors 302, 304, 306 is processed using a number of available algorithms, represented by the block 308. These algorithms may be implemented, for example, via suitable programming of the apparatus 200, whereby the necessary executable instructions are stored within the memory 210, and executed by the microprocessor 202. Resulting TR images are transmitted 310 for presentation to a human user 312. As noted above, this presentation may be via an implanted prosthetic device. For experimental purposes, the inventors have successfully implemented the algorithms described hereafter for display to a sighted human user 312 via a head-mounted display (HMD) unit.

A user input signal 314 is available to control the operation of the algorithms 308. In particular, the user input 314 may be employed to select one or more TR algorithms to be executed and blended into the final transformed image that is rendered to the human user 312.

A first exemplary TR algorithm that may be implemented within the visual processing system 300 is a structural edge detection algorithm, as illustrated by the flowchart 400 in Figure 4.

The use of edge detection has been previously proposed as a possible image processing step for bionic vision, with the goal of simplifying the visual scene to a line-drawing-like picture. The use of Canny edge detection, which finds edges by analysis of two-dimensional image data only, has been trialled, unsuccessfully, in offline simulated prosthetic vision tests (i.e. wherein static images are preprocessed then presented to users as phosphene patterns).

The concept of simplifying a scene by an edge-based representation is useful if structural edges, i.e. edges that mirror line drawings of three-dimensional objects, are used to represent the scene. Humans regularly use two-dimensional drawings to convey three-dimensional information. Indeed, many optical illusions operate based on the inability of human vision to reject three-dimensional information perceived from two-dimensional drawings. To make the most of low resolution bionic vision, it is desirable to encode a three-dimensional world into a sparse two-dimensional line drawing, and to present the user with a set of dots that represents structurally salient lines of a visual scene. The algorithm 400 implements a structural edge detector that operates on a depth image instead of the traditional approach of two-dimensional visual edge detection. By using a depth sensor, 'edge noise' caused by visual textures and visible illumination such as shadows may be avoided.

A structural edge may be defined as a location at which there is a sufficiently non-planar region in depth (i.e. distance from the depth sensor), where the region is defined as a contiguous patch of depth values. According to this definition, a flat or gently curving surface such as a wall or table generates no edges whereas any anomaly such as the table edge or objects protruding from the table will produce edges. An additional benefit is locations where there is a "crease" edge, such as wrinkles on a table cloth, will also be detected as a structural edge.

The algorithm 400 commences with the receipt of depth sensor information 402. In the system 300, this input is in the form of an array of pixels (u, v), in which each pixel value is proportional to the stereo disparity $\delta$ governed by the formula:

$$\delta = \frac{1}{z} = \lambda_a \cdot Depth(u, v) + \lambda_b$$

where z is the metric distance of the object from the depth sensor, and the parameters $\lambda_a$ and $\lambda_b$ are characteristic of the particular sensor, and are initially determined via a simple calibration process.

At step 404, the input data is processed in order to adapt it to the further processing steps. In the exemplary embodiment, the raw depth image from the sensor is resized to 175x175 pixels. (This results in a change in aspect ratio, which is ignored because empirical tests have shown that it has little impact on the structural edge detection results.) The resized depth image is adapted to the 25x25 pixel target output image, and at step 406 is segmented a 25x25 array of 'patches' of 7x7 pixels, each of which contains 49 raw depth values proportional to stereo disparity $\delta$.

Processing at step 408 is performed using the disparity-based depth values, which exhibits better error characteristics (isotropic relative to distance) and incurs less computational cost that distance-based processing. This processing comprises analysis of the 7x7 segments to detect significant discontinuities in distance, representing structural edges.

In the exemplary embodiment, processing 408 comprises performing principal component analysis (PCA) of each depth pixel patch, resulting in three eigenvalues and their corresponding eigenvectors. For a patch where the three-dimensional structure is coplanar, the first two eigenvalues will be high as their eigenvectors will be parallel to the plane (pointing in orthogonal directions) whereas the third eigenvalue will be zero since there is no variance in the direction perpendicular to the plane. As the depth data deviates from a plane, the third eigenvalue will increase in size. By applying a suitable predetermined threshold to the third eigenvalue, significant discontinuities and crease edges within the patch are identified. The threshold can be varied to allow for a range of sensitivities when detecting structural edges. A calibration may be performed, e.g. using test environments, to determine a suitable threshold corresponding with a 'significant' discontinuity, i.e. one which would be interpreted by a sighted person as a structural edge.

At step 410, the detected discontinuities are rendered as phosphenes (pixels) in the transformed image output.

Figure 5 illustrates the results of application of the exemplary algorithm 400. A real-world scene 502 includes a patterned tablecloth, a white bowl, and books. A corresponding downsampled visual image 504 of the scene 502 retains almost no salient information regarding the objects. This shows that the traditional approach of downsampling followed by binary thresholding only represents parts of the white bowl and the specular reflection of the books. Moreover, the patterned table cloth results in noisy edges. In particular, the large number of high gradient locations caused by the textured table cloth will flood low resolution downsampled images with a large number of edges. Moreover, the selection of thresholds and the scale of edge detection is difficult without *a priori* knowledge of the visual scene. By using a depth seonsor, the detection of structural edges is performed, as illustrated in image 506, to allow concise scene representation as shown by the final transformed image 508. The improvement in the saliency of visual information presented for the objects on the table a clearly apparent.

A second exemplary TR algorithm that may be implemented within the visual processing system 300 is a ground plane detection algorithm, as illustrated by the flow diagram 600 in Figure 6.

The goal of ground plane detection is to provide rendering of 'clear space' in front of a user. 'Clear space' corresponds with those parts of the scene that belong to the three-dimensional structure of the ground plane. By employing a depth sensor, ground plane detection may be highly robust, and be unaffected by the visual appearance of the ground. This allows an accurate rendering of the ground plane in realistic environments, including low contrast and spatially complex scenes.

As shown in Figure 6, in addition to the depth sensor input 602, input 604 is received from a three-axis accelerometer. Use of the accelerometer data greatly improves the initial accuracy of the algorithm and reduces subsequent computational complexity.

The algorithm 600 generates, within block 606, multiple 'plane hypotheses' offset in the direction of gravity to accommodate users of different heights. The best plane hypothesis 608 is refined using RANdom SAmple Consensus (RANSAC) 610. The depth image locations of ground plane inliers of the RANSAC-refined plane are rendered 612 as a 25x25 transformed image, suitable for a bionic vision system.

Plane hypotheses are rapidly generated by taking advantage of accelerometer readings. The direction of gravity is estimated by taking the temporal running average over three consecutive accelerometer readings to smooth away jitters caused by sensor noise and user movements. Alternatively, an information filter such as Kalman Filtering can be used to smooth sensor noise. This smoothed gravity vector $\hat{x}g$ is used to directly estimate the normal of the ground plane. To prevent the incorrect detection of flat objects like tables as the ground plane, a camera-to-plane offset D is set to a range of discrete heights $H_i$. Plane hypotheses are generated according to (in Euclidean coordinates):

$$Ax + By + Cz + D = 0$$

with

$$A = -g_x \quad B = -g_y \quad C = g_z \quad D = H_i$$

where $(g_x, g_y, g_z)$ are components of the gravity vector (normalised to unity). The negative signs convert from the accelerometer's coordinate frame to the world coordinate frame, and invert the direction of gravity to point the plane normal upwards. In the exemplary embodiment, values of $H_i$ are selected to generate multiple plane hypotheses centered around the user's standing height.

The exemplary algorithm performs ground plane fitting using disparity, which has the benefits of isotropic error with increasing distance (i.e. errors are greater at greater distances, where their impact is less significant) and low computational cost in calculating the metric locations of each depth pixel as three-dimensional points. Computational savings are also made when rendering, because the resulting plane fit is computed directly in the depth image. As such, the Euclidean three-dimensional plane model is converted to the corresponding disparity model:

$$\delta = \alpha u + \beta v + \gamma$$

where $\delta$ is the disparity and $(u,v)$ are image coordinates. The disparity plane parameters are defined as follows:

$$\alpha = -A.L/D \quad \beta = -B.L/D \quad \gamma = -C.Lf/D$$

where L is the baseline distance between the depth sensor and infrared projector (7.5 cm) and f is the focal length in pixels (515).

The best plane hypothesis 608 is defined as the one with the most inliers. Outliers are detected according to the following condition, which measures deviation from the ideal plane:

$$\delta(u,v) > \alpha u + \beta v + \gamma + \delta_0$$

As the accelerometer only provides a rough estimate of the plane normal, a liberal threshold of $\delta_0 = 2$ pixels is employed to ensure sufficient inlier support. The best plane hypothesis, along with its inliers, is passed onto the RANSAC refinement step 610.

The best plane hypothesis up to this point assumes that the plane normal can be measured by the accelerometer (i.e. via the direction of gravity g). Due to sensor noise, user movement and imperfections of the physical ground plane, this assumption may not be robust enough for real-world use. To remedy this, the disparity plane parameters are refined using RANSAC. In each RANSAC iteration, three points are sampled from the depth image to generate a disparity plane estimate. The plane parameters are found by solving the following linear system, where $(u,v,\delta)$ are the image coordinates and disparity sampled from the depth image:

$$X \begin{pmatrix} \alpha \\ \beta \\ \gamma \end{pmatrix} = \begin{pmatrix} \delta_1 \\ \delta_2 \\ \delta_3 \end{pmatrix}$$

where

$$X = \begin{pmatrix} u_1 & v_1 & 1 \\ u_2 & v_2 & 1 \\ u_3 & v_3 & 1 \end{pmatrix}$$

The inliers from the accelerometer-based plane hypothesis are checked against the RANSAC estimate at each iteration using the outlier condition with a lower threshold of $\delta_0 = 0.3$. This ensures a more accurate plane fit. The plane with the most inliers over 10 iterations of RANSAC is the detected ground plane.

Setting of the RANSAC disparity threshold to a low value has the side effect of excluding ground plane depth pixels that deviate from the RANSAC plane estimate due to sensor noise. The use of an accelerometer-based approach to produce the input plane hypothesis and inliers may also exclude ground plane pixels due to small perturbations in the estimated gravity vector. These issues will result in gaps in the rendered ground plane. To overcome this problem in the rendering step 612, all depth image pixels are compared against the final RANSAC plane estimate using a threshold of $\delta_0 = 0.8$. Inliers in the depth image are thresholded and resized using Gaussian pyramids to produce a transformed image of 25x25 pixels. The use of Gaussian pyramids prevents aliasing, which is crucial due to the low fidelity of bionic vision. The 25x25 binary image may then be rendered as a phosphene pattern.

Figure 7 illustrates the results of application of the exemplary algorithm 600. A real-world scene 702 includes a number of items of furniture, a tiled floor, and a standing person. A corresponding downsampled image 704 of the scene 702 retains almost no salient information regarding the location of the ground plane. The tiled flooring and dense constellations of obstacles make a direct visual representation of the scene bear little resemblance to the salient structure that would guide a user through the open space during visual navigation. It is difficult to imagine how a binary thresholding process can produce a coherent image for such a visually complex scene. By contrast, the ground plane is readily identified using the depth sensor data, as illustrated by image 706, and can be rendered effectively despite the presence of the contrasting tiles, as shown by the final transformed image 708.

A third exemplary TR algorithm that may be implemented within the visual processing system 300 is a face and body detection algorithm, as illustrated by the flow diagram 800 in Figure 8.

Inputs to the face and body detection algorithm 800 are a two-dimensional visual image 802 (which may be monochrome), and a corresponding depth image 804. A boosted Haar cascade algorithm 806 is used to perform frontal face detection on the monochrome image 802. Each detected face is returned as a bounding rectangle in image coordinates. Multiple faces can be detected from a single image.

The body below each face is found by performing a proximity search in the depth image. Each depth pixel is converted into a metric (x,y,z) 3D location as follows:

$$x = \frac{(u - c_x)z}{f_x} \quad , \quad y = \frac{(v - c_y)z}{f_y} \quad , \quad z = \frac{1}{\lambda_a \cdot Depth(u,v) + \lambda_b}$$

where $(f_x, f_y)$ are the focal lengths, $(c_x, c_y)$ are the principal point offsets and (u,v) are the pixel coordinates of the depth sensor.

Depth pixels with a three-dimensional location within a cylindrical volume below the face are retained while the rest are cleared. The largest 8-connected component *blob* is considered to be the body segment attached to the face. This process is repeated for each detected face, which allows the segmentation of multiple bodies.

Low resolution icons or avatars are used to represent frontal faces as visual and structural representation of the face based on sensor data is difficult. This allows a symbolic representation of the detected face registered to the body segment detected using the depth camera. The face icons and body segments are combined in step 810, as follows.

Transformed image output 812 suitable for bionic vision is rendered by first drawing the body segment in low resolution using the same Gaussian pyramidal down sampling approach as described above for rendering the ground plane. The face icon with a size that matches the detected face is then drawn over the body segment. This process is repeated for each detected face, which allows the representation of multiple people.

Figure 9 illustrates the results of application of the exemplary algorithm 800. A real-world scene 902 includes two people, where the one on the left is waving. Blind people miss out on the visual cues we take for granted when interacting with other human beings, including the pose (location, orientation, expression) of a person's face and the person's body (posture and gestures). No improvement is offered by the conventional downsampling approach, shown in image 904, and the structural edges algorithm, represented by the image 906, produces an overly-complex image in which features of interest are swamped by features of low salience, such as folds and ripples in clothing.

The face and body detection algorithm 800 seeks to provide fundamental visual cues that will help improve human interactions. The improvements of having a dedicated algorithm for face and body detection can be seen in images 908-912. In particular, the image 908 illustrates the bounding boxes resulting from face detection 806. Image 910 illustrates the outlines of the bodies identified by body segmentation 808. The final transformed image 912 shows the clarity with which people may be represented in compared with traditional bionic vision 904 and structural edges 906.

In many real-world situations, a bionic vision user may wish to select between TR transformation and/or to enable multiple TR transformations at once. Embodiments of the invention therefore provide for user input to enable such switching and selection.

For example, blending the results of the ground plane and structural edges algorithms may provide navigational assistance in complex environments, and allow the user to identify objects. To accommodate this need, an automatic blending algorithm may be implemented, and exemplary embodiment of which is illustrated in the flow chart 1000 of Figure 10. The algorithm 1000 blends the outputs from multiple transformation modes into a single low resolution output.

The algorithm commences at step 1002, with input of two or more transformed images, e.g. 25x25 pixel TR outputs in the described embodiment. The received images are assigned a priority order, either explicitly or implicitly based in content (step 1004).

At step 1006 blending is performed of the two lowest-priority 25x25 TR outputs by using saturation arithmetic, where values are limited to ON or OFF. For example, an ON pixel added to an ON pixel remains ON and the same rule applies for OFF minus OFF. This advantageously enables rapid real-time blending at minimal computational cost. In an exemplary embodiment, rendering of face and body images has precedence over rendering of ground plane images, which in turn has precedence over rendering of structural edges. This is because detected human beings will generally be in the foreground of the spatial field, rendering of the ground plane is important to enable safe navigation within the spatial field, while structural edged will typically be located adjacent to, or outside, the region occupied by the ground plane. There is accordingly an implicit priority of, in ascending order, structural edges, ground plane and face/body.

Decision point 1008 determines whether there are higher-priority images still to be blended and, if so, control returns to step 1006.

In the exemplary embodiment, the two lowest-priority outputs, i.e. ground plane detection (G) and structural edges (E), are blended using the following equation:

$$blend(G,E) = E - dilate(G) + G$$

where the 'dilate()' function performs morphological dilation by one-pixel using a 3x3 kernel.

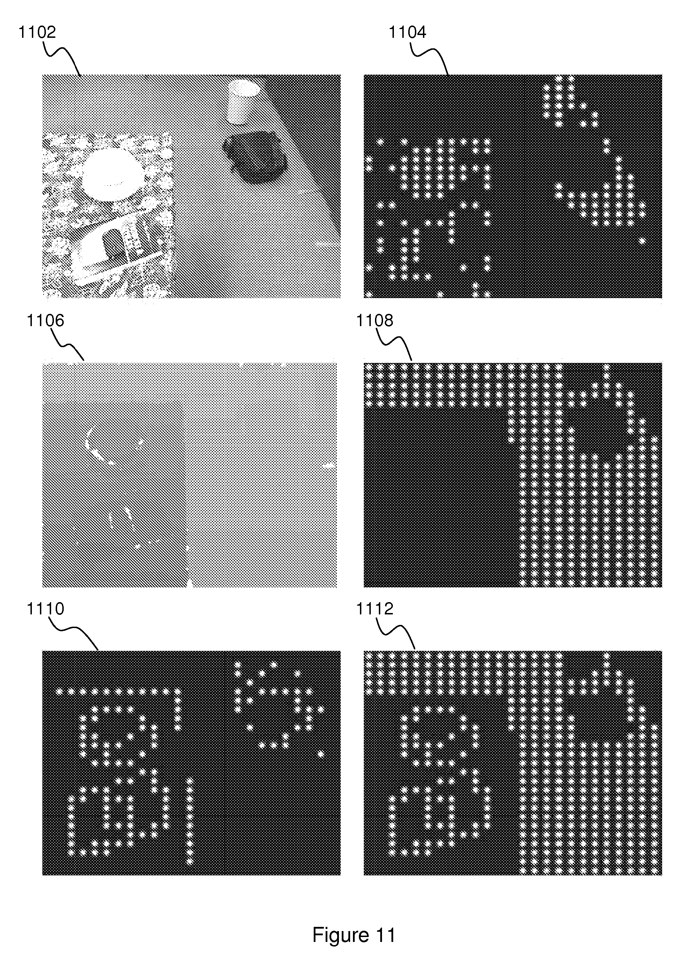

Figure 11 illustrates the effectiveness of blending ground plane and structural edges transformed images. A real-word scene 1102 includes a clear area of floor, a table with objects resting on its surface, and obstacles located on the floor. The conventional downsampled image 1104 provides minimal salient information of the physical structure of the space. In combination with the corresponding depth image 1106, and accelerometer readings, the ground plane 1108 and structural edges 1110 transformed images are generated. The blended output 1112 includes clear salient features corresponding with the objects and obstacles in the scene 1102, and of the navigable floor area.

In a subsequent iteration, face and body (F) is blended into the resulting image:

$$blend(F, blend(G,E)) = blend(G,E) - dilate(F) + F$$

As will be appreciated, an iterative approach 1000 is only one possible implementation of this algorithm. A direct blending of the three transformed images (E, G, F) can be represented by a single equation, and implemented in a variety of ways:

$$blend(G,E,F) = (E - dilate(G) + G) - dilate(F) + F$$

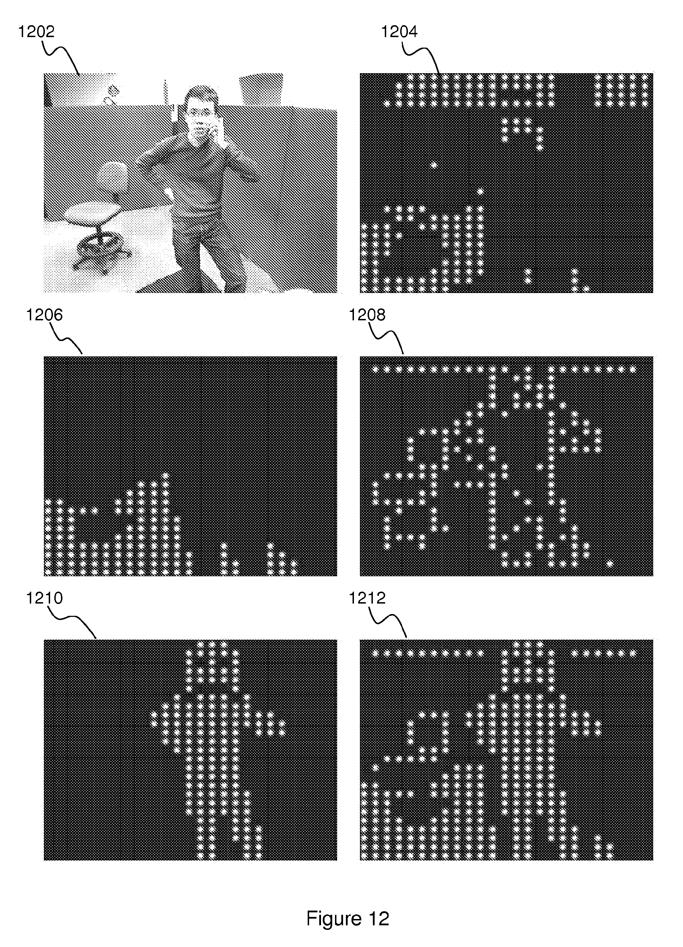

Figure 12 illustrates the effectiveness of blending face and body with the ground plane and structural edges transformed images. A real-word scene 1202 includes a person and a chair standing on a clear area of floor. The conventional downsampled image 1204 again provides minimal salient information of the physical structure of the space, the location, posture, or other features of the person or chair. Ground plane 1206, structural edges 1208 and face and body 1210 are blended to produce the combined image 1212 of the scene. The location and posture of the person, the chair and the floor are all clearly visible in this image.

The foregoing description of particular embodiments of the invention is provided by way of example only. Numerous variations and modification will be apparent to those skilled in the relevant art. Accordingly, the embodiments are not to be considered limiting of the scope of the invention, which is as defined in the claims appended hereto.

**Claims**

1. A prosthetic processing apparatus (200), the apparatus comprising:

    a visual sensor (104) configured to output
    two-dimensional image information as physical information of a spatial field; and
    an output interface (208) coupled to a sensory input device which is configured to apply a signal (114) to a visual sensory pathway of a visually impaired subject,
    the apparatus further comprises: at least one additional sensor (106) configured to output physical information other than two-dimensional image information of the spatial field; and
    a processor (202) operatively coupled (204, 206) to the sensors and to the output interface (208), and which is configured to:

        receive the physical information of the spatial field from the sensors;
        process the received information to identify one or more salient features of a predetermined category within the spatial field;
        generate a transformed representation of the spatial field in which each identified salient feature is represented in a symbolic form subject to predetermined fidelity constraints imposed by capability of the sensory input device, said transformed representation being an image; and
        output the transformed representation to the sensory input device via the output interface.

2. The apparatus of claim 1 wherein the additional sensor (106) comprises one or more of a depth sensor, and an accelerometer.

3. The apparatus of claim 1 wherein the additional sensor (106) comprises a depth sensor, and the processor (202) is configured to apply a structural edge-detection algorithm (400) whereby physical information received from the depth sensor is processed to identify locations at which discontinuities in depth are detected.

4. The apparatus of claim 1 wherein the processor (202) is configured to apply a face detection algorithm to two-dimensional image information received from the visual sensor (104) in order to identify the location of faces within the image.

5. The apparatus of claim 4 wherein the additional sensor (106) comprises a depth sensor, and the processor (202) is configured to apply a body detection algorithm (800) whereby physical information received from the depth sensor is processed to identify physical configuration of human bodies associated with located faces.

6. The apparatus of claim 5 wherein the body detection algorithm (800) comprises a proximity search (808) of depth sensor information to identify features falling within a specified volume in the vicinity of located faces.

7. The apparatus of claim 1 wherein the additional sensor (106) comprises an accelerometer and the processor is configured to estimate a direction of gravity based upon physical information received from the accelerometer, whereby a spatial orientation of physical information received from one or more additional sensors is determined.

8. The apparatus of claim 1 wherein the additional sensor (106) comprises a depth sensor and an accelerometer, and wherein the processor is configured to apply a ground plane detection algorithm (600) whereby physical information received from the depth sensor is processed along with physical information received from the accelerometer in order to identify locations corresponding with a contiguous substantially horizontal plane surface with the spatial field.

9. The apparatus of claim 8 wherein the processor (202) is configured to apply the ground plane detection algorithm (600) which comprises:

    generating a plane hypothesis corresponding with a hypothetical plane disposed at a predetermined elevation relative to the depth sensor;
    testing the plane hypothesis by comparing a distance measure of points within the spatial field detected by the depth camera with points on the hypothetical plane; and

accepting the plane hypothesis in the event that the comparison establishes a sufficiently close correlation between the detected points and the points on the hypothetical plane.

10. The apparatus of claim 9 wherein the ground plane detection algorithm (600) further comprises generating (606) multiple plane hypotheses corresponding with hypothetical planes disposed at a plurality of predetermined elevations relative to the depth sensor; and
accepting the plane hypothesis having the closest correlation between the detected points and the points on the hypothetical plane.

11. The apparatus of claim 9 wherein physical information received from the accelerometer is used for determining a direction normal to the hypothetical ground plane, and wherein the ground plane detection algorithm (600) includes estimating the location of the horizontal plane surface by determining an improved plane estimate by applying an iterative method (610) based upon sampling of depth sensor information corresponding with points within the accepted hypothetical plane.

12. The apparatus of claim 1 wherein the processor (202) is configured to apply a blending algorithm (1000) to generate a transformed representation of the spatial field comprising elements of corresponding representations produced by two or more transformative algorithms, and wherein the blending algorithm assigns a precedence to the representations produced by the two or more transformative algorithms, in a manner that results in the most effective presentation of salient information.

13. A visual processing method comprising:

receiving information from a plurality of sensors (104, 106) configured to capture and output physical information of a spatial field,
wherein the plurality of sensors comprises:
a visual sensor (104) configured to output two-dimensional image information of a spatial field; and
at least one additional sensor (106) configured to output physical information other than two-dimensional image information of the spatial field;
processing the received information to identify one or more salient features of a predetermined category within the spatial field;
generating a transformed representation of the spatial field in which each identified salient feature is represented in a symbolic form subject to predetermined fidelity constraints imposed by capability of a sensory input device configured to apply a signal to a visual sensory pathway of a visually impaired
subject, said transformed representation being an image; and
outputting the transformed representation to the sensory input device.

14. The method of claim 13 wherein the output representation is communicated to a prosthetic implant arranged to apply electrical stimulation corresponding with the transformed representation to a visual pathway of the subject.

**Patentansprüche**

1. Prothetische Verarbeitungsvorrichtung (200), wobei die Vorrichtung umfasst:

einen optischen Sensor (104), gestaltet zum Ausgeben von zweidimensionalen Bildinformationen als physikalische Informationen eines räumlichen Felds; und
eine Ausgabeschnittstelle (208), gekoppelt mit einer sensorischen Eingabevorrichtung, die dafür gestaltet ist, ein Signal (114) an einen optischsensorischen Signalweg eines sehbehinderten Subjekts anzulegen, wobei die Vorrichtung ferner umfasst:

wenigstens einen zusätzlichen Sensor (106), gestaltet zum Ausgeben von physikalischen Informationen, die von zweidimensionalen Bildinformationen des räumlichen Felds verschieden sind; und
einen mit den Sensoren und mit der Ausgabeschnittstelle (208) funktionsfähig gekoppelten (204, 206) Prozessor (202), der dafür gestaltet ist:

die physikalischen Informationen des räumlichen Felds von den Sensoren zu empfangen;
die empfangenen Informationen zu verarbeiten, um ein oder mehrere hervorstechende Merkmale einer vorbestimmten Kategorie in dem räumlichen Feld zu identifizieren;

eine transformierte Darstellung des räumlichen Felds zu erzeugen, in der jedes identifizierte hervorstechende Merkmal in einer symbolischen Form dargestellt ist, die vorbestimmten Beschränkungen der Wiedergabetreue unterworfen ist, die durch die Leistungsfähigkeit der sensorischen Eingabevorrichtung bestimmt sind, wobei die transformierte Darstellung ein Bild ist; und

Ausgeben der transformierten Darstellung an die sensorische Eingabevorrichtung über die Ausgabeschnittstelle.

2. Vorrichtung gemäß Anspruch 1, wobei der zusätzliche Sensor (106) eines oder mehrere von einem Tiefensensor und einem Beschleunigungsmesser umfasst.

3. Vorrichtung gemäß Anspruch 1, wobei der zusätzliche Sensor (106) einen Tiefensensor umfasst und der Prozessor (202) dafür gestaltet ist, einen strukturellen Kantennachweisalgorithmus (400) auszuführen, wobei von dem Tiefensensor empfangene physikalische Informationen verarbeitet werden, um Orte zu identifizieren, an denen Diskontinuitäten der Tiefe nachgewiesen werden.

4. Vorrichtung gemäß Anspruch 1, wobei der Prozessor (202) dafür gestaltet ist, einen Gesichtserkennungsalgorithmus an von dem optischen Sensor (104) empfangenen zweidimensionalen Bildinformationen anzuwenden, um den Ort von Gesichtern in dem Bild zu identifizieren.

5. Vorrichtung gemäß Anspruch 4, wobei der zusätzliche Sensor (106) einen Tiefensensor umfasst und der Prozessor (202) dafür gestaltet ist, einen Körpererkennungsalgorithmus (800) auszuführen, wobei von dem Tiefensensor empfangene physikalische Informationen verarbeitet werden, um die physische Konfiguration von menschlichen Körpern, die mit lokalisierten Gesichtern verbunden sind, zu identifizieren.

6. Vorrichtung gemäß Anspruch 5, wobei der Körpererkennungsalgorithmus (800) eine Umgebungssuche (808) von Tiefensensorinformationen zum Identifizieren von Merkmalen umfasst, die in ein bestimmtes Volumen in der Umgebung von lokalisierten Gesichtern fallen.

7. Vorrichtung gemäß Anspruch 1, wobei der zusätzliche Sensor (106) einen Beschleunigungsmesser umfasst und der Prozessor dafür gestaltet ist, die Richtung der Schwerkraft auf der Grundlage von physikalischen Informationen, die von dem Beschleunigungsmesser empfangen werden, abzuschätzen, wobei die räumliche Orientierung von physikalischen Informationen, die von einem oder mehreren zusätzlichen Sensoren empfangen werden, bestimmt wird.

8. Vorrichtung gemäß Anspruch 1, wobei der zusätzliche Sensor (106) einen Tiefensensor und einen Beschleunigungsmesser umfasst und wobei der Prozessor dafür gestaltet ist, einen Grundebenen-Erkennungsalgorithmus (600) auszuführen, wobei von dem Tiefensensor empfangene physikalische Informationen zusammen mit von dem Beschleunigungsmesser empfangenen physikalischen Informationen verarbeitet werden, um Orte zu identifizieren, die einer durchgehenden, im Wesentlichen waagrechten, ebenen Oberfläche mit dem räumlichen Feld entsprechen.

9. Vorrichtung gemäß Anspruch 8, wobei der Prozessor (202) dafür gestaltet ist, den Grundebenen-Erkennungsalgorithmus (600) auszuführen, der umfasst:

Erzeugen einer Ebenenhypothese, die einer hypothetischen Ebene entspricht, die in einer vorbestimmten Höhe relativ zu dem Tiefensensor angeordnet ist;

Prüfen der Ebenenhypothese durch Vergleichen eines Abstandsmaßes von Punkten innerhalb des von der Tiefenkamera nachgewiesenen räumlichen Felds mit Punkten auf der hypothetischen Ebene; und

Annehmen der Ebenenhypothese in dem Fall, dass der Vergleich eine ausreichend enge Korrelation zwischen den nachgewiesenen Punkten und den Punkten auf der hypothetischen Ebene ergibt.

10. Vorrichtung gemäß Anspruch 9, wobei der Grundebenen-Erkennungsalgorithmus (600) ferner das Erzeugen (606) mehrerer Ebenenhypothesen umfasst, die hypothetischen Ebenen entsprechen, die in einer Vielzahl von vorbestimmten Höhen relativ zu dem Tiefensensor angeordnet sind; und

Annehmen der Ebenenhypothese mit der engsten Korrelation zwischen den nachgewiesenen Punkten und den Punkten auf der hypothetischen Ebene.

11. Vorrichtung gemäß Anspruch 9, wobei von dem Beschleunigungsmesser empfangene physikalische Informationen zum Bestimmen einer Richtung normal auf die hypothetische Grundebene verwendet werden und wobei der Grundebenen-Erkennungsalgorithmus (600) das Abschätzen des Orts der waagrechten, ebenen Oberfläche durch Bestimmen einer verbesserten Ebenenabschätzung durch Ausführen eines iterativen Verfahrens (610) auf der Grundlage von Zusammenfassen von Tiefensensorinformationen, die Punkten in der angenommenen hypothetischen Ebene entsprechen, umfasst.

12. Vorrichtung gemäß Anspruch 1, wobei der Prozessor (202) dafür gestaltet ist, einen Mischalgorithmus (1000) auszuführen, um eine transformierte Darstellung des räumlichen Felds zu erzeugen, das Elemente von entsprechenden, von zwei oder mehr Umwandlungsalgorithmen erzeugten Darstellungen umfasst, und wobei der Mischalgorithmus den von den zwei oder mehr Umwandlungsalgorithmuen erzeugten Darstellungen auf eine Weise eine Rangordnung zuordnet, die die wirkungsvollste Darstellung hervorstechender Merkmale ergibt.

13. Optisches Verarbeitungsverfahren, umfassend:

Empfangen von Informationen von einer Vielzahl von Sensoren (104, 106), die dafür gestaltet sind, physikalische Informationen eines räumlichen Felds aufzunehmen und auszugeben,
wobei die Vielzahl von Sensoren umfasst:

einen optischen Sensor (104), gestaltet zum Ausgeben von zweidimensionalen Bildinformationen eines räumlichen Felds; und
wenigstens einen zusätzlichen Sensor (106), gestaltet zum Ausgeben von physikalischen Informationen, die von zweidimensionalen Bildinformationen des räumlichen Felds verschieden sind;
Verarbeiten der empfangenen Informationen, um ein oder mehrere hervorstechende Merkmale einer vorbestimmten Kategorie in dem räumlichen Feld zu identifizieren;
Erzeugen einer transformierten Darstellung des räumlichen Felds, in der jedes identifizierte hervorstechende Merkmal in einer symbolischen Form dargestellt ist, die vorbestimmten Beschränkungen der Wiedergabetreue unterworfen ist, die durch die Leistungsfähigkeit einer sensorischen Eingabevorrichtung bestimmt sind, gestaltet zum Anlegen eines Signals an einen visuellsensorischen Signalweg eines sehbehinderten Subjekts, wobei die transformierte Darstellung ein Bild ist; und
Ausgeben der transformierten Darstellung an die sensorische Eingabevorrichtung.

14. Verfahren gemäß Anspruch 13, wobei die Ausgabe-Darstellung an ein prothetisches Implantat kommuniziert wird, das dafür ausgelegt ist, eine elektrische Anregung, die der transformierten Darstellung entspricht, an einen visuellen Signalweg des Subjekts anzulegen.

**Revendications**

1. Appareil de traitement prosthétique (200), cet appareil comprenant :

un capteur visuel (104) configuré de façon à fournir des informations d'image bidimensionnelle comme des informations physiques d'un champ spatial ; et
une interface de sortie (208) couplée à un dispositif d'entrée sensorielle qui est configuré de façon à appliquer un signal (114) sur une voie sensorielle visuelle d'un sujet ayant une déficience visuelle,
cet appareil comprenant en outre :

au moins un capteur visuel supplémentaire (106) configuré de façon à fournir des informations physiques autres que des informations d'image bidimensionnelle du champ spatial ; et
un processeur (202) couplé de manière opérationnelle (204, 206) aux capteurs et à l'interface de sortie (208), et qui est configuré de façon à :

recevoir les informations physiques du champ spatial venant des capteurs ;
traiter les informations reçues afin d'identifier un ou plusieurs traits saillants d'une catégorie prédéterminée à l'intérieur du champ spatial ;
générer une représentation transformée du champ spatial dans laquelle chaque trait saillant identifié est représenté dans une forme symbolique sous réserve de contraintes de fidélité prédéterminées imposées par la capacité du dispositif d'entrée sensorielle, ladite représentation transformée étant une image ; et à

fournir cette représentation transformée au dispositif d'entrée sensorielle via l'interface de sortie.

2. Appareil selon la revendication 1, dans lequel le capteur supplémentaire (106) comprend un ou plusieurs capteurs de profondeur et un accéléromètre.

3. Appareil selon la revendication 1, dans lequel le capteur supplémentaire (106) comprend un capteur de profondeur, et le processeur (202) est configuré de façon à appliquer un algorithme de détection des contours structurels (400), comme quoi les informations physiques reçues du capteur de profondeur sont traitées de façon à identifier les emplacements auxquels des discontinuités de profondeur sont détectées.

4. Appareil selon la revendication 1, dans lequel le processeur (202) est configuré de façon à appliquer un algorithme de détection de visages aux informations d'image bidimensionnelle reçues du capteur visuel (104) de façon à identifier l'emplacement de visages à l'intérieur de l'image.

5. Appareil selon la revendication 4, dans lequel le capteur supplémentaire (106) comprend un capteur de profondeur, et le processeur (202) est configuré de façon à appliquer un algorithme de détection de corps (800), comme quoi les informations physiques reçues du capteur de profondeur sont traitées de façon à identifier la configuration physique de corps humains associés aux visages localisés.

6. Appareil selon la revendication 5, dans lequel l'algorithme de détection de corps (800) comprend une recherche de proximité (808) des informations du capteur de profondeur de façon à identifier des traits situés à l'intérieur d'un volume spécifié à proximité des visages localisés.

7. Appareil selon la revendication 1, dans lequel le capteur supplémentaire (106) comprend un accéléromètre et le processeur est configuré de façon à estimer une direction de gravité en se basant sur les informations physiques reçues de cet accéléromètre, comme quoi une orientation spatiale des informations physiques reçues d'un ou de plusieurs capteurs supplémentaires est déterminée.

8. Appareil selon la revendication 1, dans lequel le capteur supplémentaire (106) comprend un capteur de profondeur et un accéléromètre, et dans lequel le processeur est configuré de façon à appliquer un algorithme de détection de plan de fond (600), comme quoi les informations physiques reçues du capteur de profondeur sont traitées avec les informations physiques reçues de l'accéléromètre afin d'identifier les emplacements correspondant à une surface de plan essentiellement horizontale contiguë avec le champ spatial.

9. Appareil selon la revendication 8, dans lequel le processeur (202) est configuré de façon à appliquer l'algorithme de détection de plan de fond (600) qui comprend :

la génération d'une hypothèse de plan correspondant à un plan hypothétique disposé à une élévation prédéterminée relativement au capteur de profondeur ;
la vérification de cette hypothèse de plan en comparant une mesure de distance de points à l'intérieur du champ spatial détecté par la caméra de profondeur avec des points sur le plan hypothétique ; et
l'acceptation de l'hypothèse de plan au cas où la comparaison établit une corrélation suffisamment proche entre les points détectés et les points sur le plan hypothétique.

10. Appareil selon la revendication 9, dans lequel l'algorithme de détection de plan de fond (600) comprend en outre la génération (606) d'hypothèses de plans multiples correspondant aux plans hypothétiques disposés à une pluralité d'élévations prédéterminées relativement au capteur de profondeur ; et
l'acceptation de l'hypothèse de plan ayant la corrélation la plus proche entre les points détectés et les points sur le plan hypothétique.

11. Appareil selon la revendication 9, dans lequel les informations physiques reçues de l'accéléromètre sont utilisées pour déterminer une direction normale au plan de fond hypothétique, et dans lequel l'algorithme de détection du plan de fond (600) comprend l'estimation de l'emplacement de la surface du plan horizontale en déterminant une estimation du plan améliorée en appliquant un procédé itératif (610) basé sur l'échantillonnage des informations du capteur de profondeur correspondant à des points à l'intérieur du plan hypothétique accepté.

12. Appareil selon la revendication 1, dans lequel le processeur (202) est configuré de façon à appliquer un algorithme de mélange (1000) de façon à générer une représentation transformée du champ spatial comprenant des éléments de représentations correspondantes produites par deux algorithmes de transformation ou plus, et dans lequel cet algorithme de mélange attribue une précédence aux représentations produites par les deux algorithmes de transformation ou plus, d'une manière qui résulte en la présentation la plus efficace d'informations saillantes.

13. Procédé de traitement visuel comprenant :

la réception d'informations d'une pluralité de capteurs (104, 106) configurés de façon à capturer et à fournir des informations physiques d'un champ spatial,
dans lequel la pluralité de capteurs comprend :

un capteur visuel (104) configuré de façon à fournir des informations d'image bidimensionnelle d'un champ spatial ; et
au moins un capteur visuel supplémentaire (106) configuré de façon à fournir des informations physiques autres que des informations d'image bidimensionnelle du champ spatial ;
le traitement des informations reçues afin d'identifier un ou plusieurs traits saillants d'une catégorie prédéterminée à l'intérieur du champ spatial ;
la génération d'une représentation transformée du champ spatial dans laquelle chaque trait saillant identifié est représenté dans une forme symbolique sous réserve de contraintes de fidélité prédéterminées imposées par la capacité d'un dispositif d'entrée sensorielle configuré de façon à appliquer un signal sur une voie sensorielle visuelle d'un sujet ayant une déficience visuelle, ladite représentation transformée étant une image ; et
la fourniture de cette représentation transformée au dispositif d'entrée sensorielle.

14. Procédé selon la revendication 13, dans lequel la représentation de sortie est communiquée à un implant prosthétique agencé de façon à appliquer une stimulation électrique correspondant à la représentation transformée sur une voie visuelle du sujet.

Figure 1

Figure 2

300

302

Kinect

Colour video

304 — Depth video

Accelerometer

306

@30Hz

308

**Intelligent algorithms**

310

Ground plane

Structural edges

Face and body

312

**Human User**

**Mode toggle**

314

Figure 3

400

Receive depth sensor input          402

Segment sensor data          404

Analyse to detect depth changes          406

Render transformed image          408

Figure 4

Figure 5

Figure 6

702

704

706

Figure 7

708

802

806

810

Monochrome image

Face detection using boosted haar cascades

Faces

Combine face avatar and body contour

Render to bionic vision

812

Faces

Depth image

Find 3D points within cylindrical volume below each face

Contour of body

800

804

808

Figure 8

902

904

906

908

910

912

Figure 9

1000

Receive two or more transformed images —1002

Order images according to priority —1004

Blend two lowest priority images —1006

1008

Yes — More?

No

Figure 10

1102

1104

1106

1108

1110

1112

Figure 11

1202

1204

1206

1208

1210

1212

Figure 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008109771 A
- GB 2477431 A

- US 6658299 B